(19) Europäisches Patentamt
European Patent Office
Office européen des brevets

(11) Veröffentlichungsnummer: **0 407 790 A1**

(12) # EUROPÄISCHE PATENTANMELDUNG

(21) Anmeldenummer: **90111937.0**

(22) Anmeldetag: **23.06.90**

(51) Int. Cl.5: **C07D 207/34**, C07D 405/04, A01N 43/36

(30) Priorität: **05.07.89 DE 3922104**

(43) Veröffentlichungstag der Anmeldung: **16.01.91 Patentblatt 91/03**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Heinemann, Ulrich, Dr.**
**Feldstrasse 18**
**D-6553 Leichlingen 1(DE)**
Erfinder: **Wollweber, Detlef, Dr.**
**Paul-Ehrlich-Strasse 17**
**D-5600 Wuppertal 1(DE)**
Erfinder: **Brandes, Wilhelm, Dr.**
**Eichendorffstrasse 3**
**D-5653 Leichelingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **N-Vinyl-3-cyano-4-phenyl-pyrrol-Derivate.**

(57) Neue N-Vinyl-3-cyano-4-phenyl-pyrrol-Derivate der Formel (I)

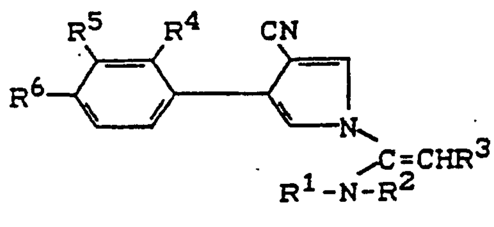

(I)

in welcher
$R^1$ für Alkyl steht,
$R^2$ für Alkyl steht oder
$R^1$ und $R^2$ gemeinsam mit dem Stickstoffatom, an welches sie gebunden sind, einen 5- bis 7-gliedrigen gesättigten, gegebenenfalls gleich oder verschieden, ein- bis mehrfach substituierten Ring bilden,
$R^3$ für Alkyl steht,
$R^4$ für Wasserstoff, Halogen, Alkyl, Halogenalkyl, Alkoxy, Halogenalkoxy, Alkylthio oder Halogenalkylthio steht,
$R^5$ für Wasserstoff, Halogen, Alkyl, Halogenalkyl, Alkoxy, Halogenalkoxy, Alkylthio oder Halogenalkylthio stehen und
$R^4$ und $R^5$ gemeinsam für durch Halogen substituiertes Alkylendioxy stehen und

EP 0 407 790 A1

$R^6$ für Wasserstoff oder Halogen steht,
ein Verfahren zu ihrer Herstellung und ihre Verwendung zur Bekämpfung von Schädlingen.

## N-VINYL-3-CYANO-4-PHENYL-PYRROL-DERIVATE

Die Erfindung betrifft neue N-Vinyl-3-cyano-4-phenyl-pyrrol-Derivate, ein Verfahren zu ihrer Herstellung und ihre Verwendung zur Bekämpfung von Schädlingen, insbesondere pflanzenschädigende Pilze.

Es ist bekannt, daß am Pyrrol-Stickstoff unsubstituierte oder substituierte 3-Cyano-4-phenyl-pyrrol-Derivate z.B. eine fungizide Wirksamkeit besitzen (vgl. z.B. EP 130 149, EP 182 737, EP 206 999, EP 236 272, GB 2 141 709, DE 3 718 375). So sind in der EP 182 737 am Pyrrolstickstoff substituierte Verbindungen beschrieben, z.B. N-Vinyl-3-cyano-4-(2,3-dichlorphenyl)-pyrrol, die fungizide Eigenschaften haben.

Die Wirksamkeit dieser vorbekannten Verbindung ist jedoch insbesondere bei niedrigen Aufwandmengen und Konzentrationen nicht in allen Anwendungsgebieten völlig zufriedenstellend.

Es wurden neue N-Vinyl-3-cyano-4-phenyl-pyrrol-Derivate der allgemeinen Formel (I),

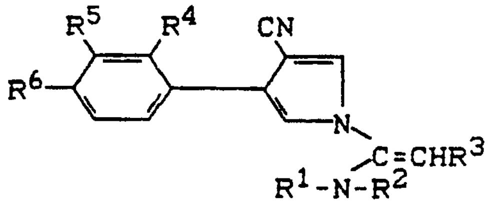

(I)

in welcher
$R^1$ für Alkyl steht,
$R^2$ für Alkyl steht oder
$R^1$ und $R^2$ gemeinsam mit dem Stickstoffatom, an welches sie gebunden sind, einen 5- bis 7-gliedrigen gesättigten, gegebenenfalls gleich oder verschieden, ein- bis mehrfach substituierten Ring bilden,
$R^3$ für Alkyl steht,
$R^4$ für Wasserstoff, Halogen, Alkyl, Halogenalkyl, Alkoxy, Halogenalkoxy, Alkylthio oder Halogenalkylthio steht,
$R^5$ für Wasserstoff, Halogen, Alkyl, Halogenalkyl, Alkoxy, Halogenalkoxy, Alkylthio oder Halogenalkylthio steht oder
$R^4$ und $R^5$ gemeinsam für durch Halogen substituiertes Alkylendioxy stehen und
$R^6$ für Wasserstoff oder Halogen steht, gefunden.

Weiterhin wurde gefunden, daß man die neuen N-Vinyl-3-cyano-4-phenyl-pyrrol-Derivate der allgemeinen Formel (I)

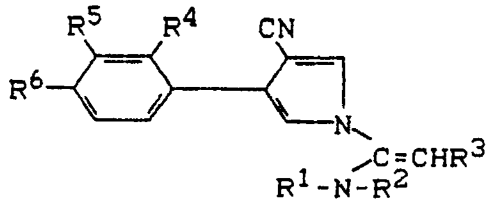

(I)

in welcher
$R^1$ für Alkyl steht,
$R^2$ für Alkyl steht oder
$R^1$ und $R^2$ gemeinsam mit dem Stickstoffatom, an welches sie gebunden sind, einen 5- bis 7-gliedrigen gesättigten, gegebenenfalls gleich oder verschieden, ein- bis mehrfach substituierten Ring bilden,
$R^3$ für Alkyl steht,
$R^4$ für Wasserstoff, Halogen, Alkyl, Halogenalkyl, Alkoxy, Halogenalkoxy, Alkylthio oder Halogenalkylthio steht,
$R^5$ für Wasserstoff, Halogen, Alkyl, Halogenalkyl, Alkoxy, Halogenalkoxy, Alkylthio oder Halogenalkylthio steht oder
$R^4$ und $R^5$ gemeinsam für durch Halogen substituiertes Alkylendioxy stehen, und
$R^6$ für Wasserstoff oder Halogen steht,

erhält, wenn man 3-Cyano-4-phenyl-pyrrol-Derivate der Formel (II)

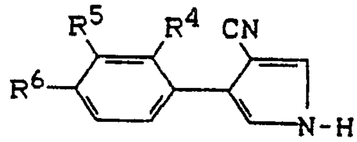

(II)

in welcher
$R^4$, $R^5$ und $R^6$ die oben angegebene Bedeutung haben,
mit 1-Aminoacetylen-Derivaten der Formel (III),

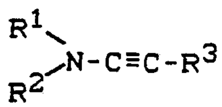

(III)

in welcher
$R^1$, $R^2$ und $R^3$ die oben angegebene Bedeutung haben,
gegebenenfalls in Gegenwart eines Verdünnungsmittels umsetzt.

Schließlich wurde gefunden, daß die neuen N-Vinyl-3-cyano-4-phenyl-pyrrol-Derivate der allgemeinen Formel (I) eine gute Wirkung gegen Schädlinge besitzen.

Bevorzugte Substituenten bzw. Bereiche der in den oben und nachstehend erwähnten Formeln aufgeführten Reste werden im folgenden erläutert:

In den allgemeinen Formeln bedeutet Alkyl vorzugsweise geradkettiges oder verzweigtes Alkyl mit 1 bis 6 Kohlenstoffatomen, bevorzugt mit 1 bis 4 und besonders bevorzugt mit 1 oder 2 Kohlenstoffatomen; beispielhaft seien genannt Methyl, Ethyl, n- und i-Propyl, n-, i-, s- und t-Butyl.

Alkoxy steht vorzugsweise als Substituent für geradkettiges oder verzweigtes Alkoxy mit 1 bis 6, besonders bevorzugt 1 bis 4 Kohlenstoffatomen je Alkylrest; beispielhaft seien genannt: Methoxy, Ethoxy, n- oder i-Propoxy, n-, i-, s- und t-Butoxy.

Halogenalkyl und Halogenalkoxy stehen bevorzugt als Substituenten für geradkettige oder verzweigte Reste mit je 1 bis 4 Kohlenstoffatomen, besonders bevorzugt mit 1 oder 2 Kohlenstoffatomen und jeweils 1 bis 9 bzw. 1 bis 5 gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluor-chlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy, Trifluorchlorethoxy, besonders hervorzuheben sind, Trifluormethyl und Trifluormethoxy.

Halogenalkylthio steht vorzugsweise als Substituent in den Resten für geradkettige oder verzweigte Reste mit je 1 bis 4 Kohlenstoffatomen, besonders bevorzugt mit 1 oder 2 Kohlenstoffatomen und jeweils 1 bis 9 bzw. 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Halogen als solches oder in den Definitionen Halogenalkyl, Halogenalkoxy und Halogenalkylthio steht für Fluor, Chlor, Brom und/oder Iod, insbesondere Fluor, Chlor und/oder Brom und besonders für Fluor oder Chlor.

Alkylthio steht vorzugsweise als Substituent für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z. B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Die erfindungsgemäßen N-Vinyl-3-cyano-4-phenyl-pyrrol-Derivate sind durch die Formel (I) allgemein definiert.

Bevorzugt sind Verbindungen der Formel (I), bei welchen

$R^1$ für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht oder

$R^1$ und $R^2$ gemeinsam mit dem Stickstoffatom, an welches sie gebunden sind, einen 5- bis 7-gliedrigen gesättigten, gegebenenfalls ein- bis fünffach, gleich oder verschieden durch Halogen, Alkyl mit 1 bis 4 Kohlenstoffatomen, Alkoxy mit 1 bis 4 Kohlenstoffatomen, Halogenalkyl oder Halogenalkoxy mit je 1 bis 4 Kohlenstoffatomen und 1 bis 9 gleichen oder verschiedenen Halogenatomen substituierten Ring bilden,

$R^3$ für geradkettiges oder verzweigtes Alkyl mit 1 bis 4 Kohlenstoffatomen steht,

$R^4$ für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkylthio mit 1 bis 4 Kohlenstoffatomen, geradkettiges oder verzweigtes Halogenalkylthio mit 1 bis 4 Kohlenstoffatomen und 1 bis 9 gleichen oder verschiedenen Halogenatomen steht,

$R^5$ für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Halogenalkylthio mit 1 bis 4 Kohlenstoffatomen und 1 bis 9 gleichen oder verschiedenen Halogentomen steht,

oder

$R^4$ und $R^5$ gemeinsam für ein- oder zweifach durch Fluor substituiertes Methylendioxy stehen und

$R^6$ für Wasserstoff oder Fluor stehen.

Besonders bevorzugt sind Verbindungen der Formel (I), bei welchen

$R^1$ für Methyl, Ethyl, n- oder i-Propyl oder n-Butyl, steht,

$R^2$ für Methyl, Ethyl, n- oder i-Propyl oder n-Butyl, steht oder

$R^1$ und $R^2$ gemeinsam mit dem Stickstoffatom, an welches sie gebunden sind, einen 5- bis 7-gliedrigen gesättigten Ring bilden,

$R^3$ für Methyl oder Ethyl steht,

$R^4$ für Wasserstoff, Fluor, Chlor, Brom, Methyl, Ethyl, Halogenalkyl mit 1 oder 2 Kohlenstoffatomen und 1 bis 5 gleichen oder verschiedenen Halogenatomen, insbesondere Fluor- und/oder Chloratomen, Methoxy, Ethoxy, Halogenalkoxy mit 1 oder 2 Kohlenstoffatomen und 1 bis 5 gleichen oder verschiedenen Halogenatomen, insbesondere Fluor- und/oder Chloratomen, Methylthio, Ethylthio, Halogenalkylthio mit 1 oder 2 Kohlenstoffatomen und 1 bis 5 gleichen oder verschiedenen Halogenatomen, insbesondere Fluor- oder Chloratomen, steht,

$R^5$ für Wasserstoff, Fluor, Chlor, Brom, Methyl, Ethyl, Halogenalkyl mit 1 oder 2 Kohlenstoffatomen und 1 bis 5 gleichen oder verschiedenen Halogenatomen, insbesondere Fluor- und/oder Chloratomen, Methoxy, Ethoxy, Halogenalkoxy mit 1 oder 2 Kohlenstoffatomen und 1 bis 5 gleichen oder verschiedenen Halogenatomen, insbesondere Fluor- und/oder Chloratomen, Methylthio, Ethylthio, Halogenalkylthio mit 1 oder 2 Kohlenstoffatomen und 1 bis 5 gleichen oder verschiedenen Halogenatomen, insbesondere Fluor- oder Chloratomen steht,

oder

$R^4$ und $R^5$ gemeinsam für zweifach durch Fluor substituiertes Methylendioxy stehen und

$R^6$ für Wasserstoff oder Fluor steht.

Ganz besonders bevorzugt sind Verbindungen der Formel (I), bei welchen

$R^1$ für Methyl oder Ethyl steht,

$R^2$ für Methyl oder Ethyl steht,

$R^3$ für Methyl oder Ethyl steht,

$R^4$ für Wasserstoff, Fluor, Chlor, Methyl, Ethyl, Trifluormethyl steht,

$R^5$ für Wasserstoff, Chlor, Methyl, Trifluormethyl steht und

$R^6$ für Wasserstoff oder Fluor steht.

Die für die Verbindungen der Formel (I) angegebenen bevorzugten Definitionen gelten auch für die Ausgangsverbindungen der Formel (II) und der Formel (III).

Im einzelnen seien außer den bei den Herstellungsbeispielen genannten Verbindungen die folgenden N-Vinyl-3-cyano-4-phenyl-pyrrol-Derivate der allgemeinen Formel (I) genannt:

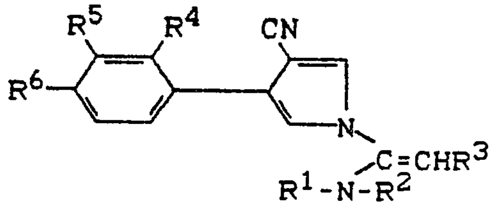

(I)

Tabelle 1:

| $R^1$ | $R^2$ | $R^3$ | $R^4$ | $R^5$ | $R^6$ |
|---|---|---|---|---|---|
| $-CH_3$ | $-CH_3$ | $-CH_3$ | F | Cl | H |
| $-C_2H_5$ | $-CH_3$ | $-C_2H_5$ | F | Cl | H |
| $-C_3H_7$-n | $-C_2H_5$ | $-CH_3$ | F | Cl | H |
| $-C_3H_7$-i | $-CH_3$ | $-CH_3$ | F | Cl | H |
| $-C_4H_9$-n | $-C_3H_7$-i | $-CH_3$ | F | Cl | H |
| $-CH_3$ | $-C_3H_7$-i | $-C_2H_5$ | F | Cl | H |
| $-CH_3$ | $C_2H_5$ | $-CH_3$ | Cl | Cl | H |
| $-C_2H_5$ | $-C_3H_7$-n | $-C_2H_5$ | Cl | Cl | H |
| $-C_2H_5$ | $-C_2H_5$ | $-CH_3$ | F | Cl | F |

Tabelle 1:

| $R^1$ | $R^2$ | $R^3$ | $R^4$ | $R^5$ | $R^6$ |
|---|---|---|---|---|---|
| $-CH_3$ | $-C_3H_7$-i | $-CH_3$ | F | Cl | F |
| $-C_2H_5$ | $-C_4H_9$-n | $-CH_3$ | Cl | Cl | F |
| $-C_2H_5$ | $-C_2H_5$ | $-CH_3$ | $-CH_3$ | $-CF_3$ | H |
| $-CH_3$ | $-C_3H_7$-i | $-CH_3$ | $-CH_3$ | $-CF_3$ | H |
| $-C_3H_7$-n | $-C_2H_5$ | $-CH_3$ | $-CH_3$ | $-CF_3$ | H |
| $-C_2H_5$ | $-CH_3$ | $-CH_3$ | $-CH_3$ | $-CH_3$ | H |
| $-CH_3$ | $-CH_3$ | $-C_2H_5$ | Cl | Cl | F |
| $-C_2H_5$ | $-C_2H_5$ | $-C_2H_5$ | $-CH_3$ | Cl | H |
| $-C_2H_5$ | $-C_3H_7$-n | $-CH_3$ | $-O-CF_2-O-$ | | H |
| $-CH_3$ | $-C_3H_7$-n | $-CH_3$ | $-O-CF_2-O-$ | | H |
| $-C_2H_5$ | $-CH_3$ | $-CH_3$ | $-O-CF_2-O-$ | | F |
| $-C_2H_5$ | $-CH_3$ | $-CH_3$ | $-CH_3$ | $-OCF_3$ | H |
| $-C_2H_5$ | $-C_2H_5$ | $-CH_3$ | $-CH_3$ | $-SCH_3$ | H |
| $-C_2H_5$ | $-C_2H_5$ | $-CH_3$ | Cl | $-OCF_3$ | H |
| $-C_2H_5$ | $-C_3H_7$-i | $-CH_3$ | $-CH_3$ | $-OCH_3$ | H |
| $-C_2H_5$ | $-CH_3$ | $-CH_3$ | F | $-OCH_3$ | F |
| $-C_2H_5$ | $-C_3H_7$-i | $-CH_3$ | $-CH_3$ | $-SCH_3$ | H |
| $-C_2H_5$ | $-CH_3$ | $-CH_3$ | F | Br | H |
| $-C_4H_9$-i | $-C_2H_5$ | $-CH_3$ | F | Br | F |
| $-C_3H_7$-i | $-C_2H_5$ | $-CH_3$ | Br | Br | H |
| $-C_4H_9$-n | $-C_2H_5$ | $-CH_3$ | F | Br | F |
| $-CH_3$ | $-C_2H_5$ | $-C_2H_5$ | Br | Br | F |

Tabelle 1: - Fortsetzung

| $R^1$ | $R^2$ | $R^3$ | $R^4$ | $R^5$ | $R^6$ |
|---|---|---|---|---|---|
| $-(CH_2)_4-$ | | $-CH_3$ | F | Cl | H |
| $-(CH_2)_4-$ | | $-CH_3$ | F | Cl | F |
| $-(CH_2)_5-$ | | $-CH_3$ | Cl | Cl | H |
| $-(CH_2)_5-$ | | $-CH_3$ | F | Cl | H |
| $-(CH_2)_5-$ | | $-C_2H_5$ | $CH_3$ | $CF_3$ | H |
| $-(CH_2)_6-$ | | $-CH_3$ | Cl | Cl | H |

Verwendet man beispielsweise 3-Cyano-4-(2-fluor-3-chlorphenyl)-pyrrol und 1-Diethylaminopropin als Ausgangsstoffe, so läßt sich der Reaktionsablauf des erfindungsgemäßen Verfahrens durch das folgende Formelschema darstellen:

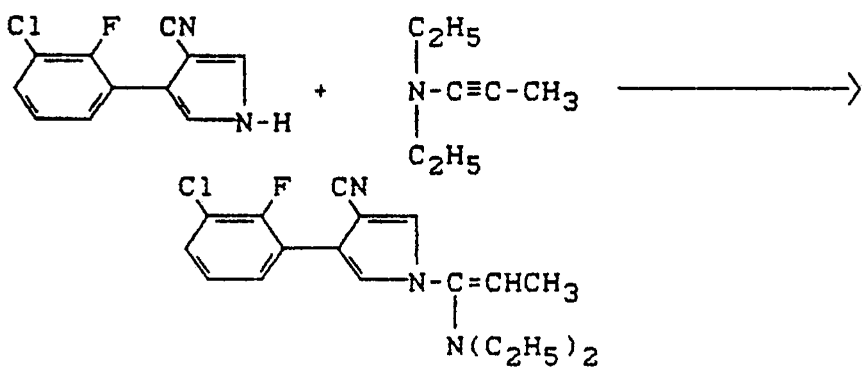

Die zur Durchführung des erfindungsgemäßen Verfahrens als Ausgangsstoffe benötigten substituierten 3-Cyano-4-phenyl-pyrrol-Derivate sind durch die Formel (II) allgemein definiert. In dieser Formel (II) stehen $R^4$, $R^5$ und $R^6$ für diejenigen Reste, die bereits im Zusammenhang mit der Beschreibung der erfindungsgemäßen Stoffe der Formel (I) für diese Substituenten genannt wurden.

Die substituierten 3-Cyano-4-phenyl-pyrrol-Derivate der Formel (II) sind zum Teil bekannt oder sind Gegenstand von nicht zum Stand der Technik gehörenden Patentanmeldungen der Anmelderin. So wird die Herstellung von 3-(2,2-Difluorbenzodioxol-4-yl)-4-cyano-pyrrol beschrieben in EP 206 999, die Herstellung von im Phenylring unsubstituierter oder durch Halogen, Alkyl oder Halogenalkyl substituierter 3-Cyano-4-phenyl-pyrrole werden in US 4 778 901, EP 174 910 beschrieben, weiterhin in DE 3 718 375, DE 3 737 984 und DE 3 718 375 u.a..

Die beim erfindungsgemäßen Verfahren zur Herstellung von Verbindungen der Formel (I) als Ausgangsstoffe zu verwendenden 1-Aminoacetylen-Derivate sind durch die Formel (III) allgemein definiert.

In Formel (III) haben $R^1$, $R^2$ und $R^3$ diejenigen Bedeutungen, die bereits oben im Zusammenhang mit der Beschreibung der erfindungsgemäßen Verbindungen der Formel (I) für $R^1$, $R^2$ und R angegeben wurden.

Die 1-Aminoacetylen-Derivate sind bekannte Verbindungen der organischen Chemie und/oder können nach an sich bekannten Verfahren hergestellt werden (vgl. Studies in Organic Chemistry, Preparative Acetylenic Chemistry Vol. 34 (2nd Edition), Elsevier 1988, S.235).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid, oder

Sulfoxide, wie Dimethylsulfoxid.

Die Reaktionstemperaturen können bei dem erfindungsgemäßen Verfahren in einem größeren Bereich variiert werden. Im allgemeinen arbeitet man bei Temperaturen zwischen - 40 °C und + 120 °C, vorzugsweise bei Temperaturen zwischen 0 °C und + 60 °C.

Zur Durchführung des erfindungsgemäßen Verfahrens setzt man pro Mol an 3-Cyano-4-phenyl-pyrrol-Derivat der Formel (II) im allgemeinen 1,0 bis 2,0 Mol, vorzugsweise 1,0 bis 1,2 Mol an 1-Aminoacetylen-Derivat der Formel (III) ein.

Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Obst- und Gemüseanbau, wie beispielsweise gegen den Erreger des Bohnengrauschimmels (Botrytis cinerea) oder zur Bekämpfung von Reiskrankheiten wie beispielsweise gegen den Erreger der Reisfleckenkrankheit (Pyricularia oryzae) oder gegen Erysiphe graminis und gegen Pyrenophora teres an Gerste einsetzen.

Weiterhin zeigen einige der erfindungsgemäßen Wirkstoffe gute fungizide Wirkungen gegen Leptosphaeria und Fusarium an Getreidekulturen. Ferner zeigen einige der erfindungsgemäßen Wirkstoffe auch eine gute und breite in vitro-Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Sohäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark po lare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

<u>Herstellungsbeispiele</u>

<u>Beispiel 1</u>

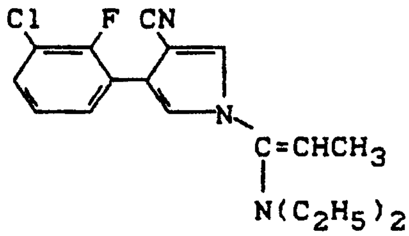

0,9 g (4 mMol) 3-Cyano-4-(2-fluor-3-chlorphenyl)-pyrrol und 0,5 g (4,5 mMol) 1-Diethylaminopropin werden in 20 ml trockenem Dichlormethan gelöst und 12 Stunden bei Raumtemperatur gerührt. Anschließend werden das Lösungsmittel und überschüssiges 1-Diethylaminopropin im Vakuum vollständig entfernt. Man erhält 1,4 g (100 % der Theorie) an N-(1-Diethylamino-2-methylvinyl)-3-cyano-4-(2-fluor-3-chlorphenyl)-pyrrol als Öl mit dem Brechungsindex $n_D^{20}$ = 1,5550.

Analog zu Beispiel 1 bzw. dem erfindungsgemäßen Verfahren können die in der nachfolgenden Tabelle angegebenen Verbindungen der Formel (I)

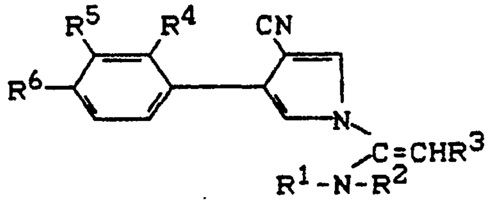

(I)

hergestellt werden:

| Bsp. Nr. | $R^1$ | $R^2$ | $R^3$ | $R^4$ | $R^5$ | $R^6$ | physikal. Daten |
|---|---|---|---|---|---|---|---|
| 2 | $-C_2H_5$ | $-C_2H_5$ | $-CH_3$ | Cl | Cl | H | $n_D^{20}$ = 1,5802 |
| 3 | $-C_2H_5$ | $-C_2H_5$ | $-CH_3$ | F | Cl | F | Fp. 92 °C |
| 4 | $-C_2H_5$ | $-C_2H_5$ | $-CH_3$ | $-CH_3$ | $-CF_3$ | H | Öl |
| 5 | $-CH_3$ | $-CH_3$ | $-CH_3$ | F | Cl | H | Öl |
| 6 | $-CH_3$ | $-CH_3$ | $-CH_3$ | Cl | Cl | H | Öl |
| 7 | $-CH_3$ | $-CH_3$ | $-CH_3$ | F | Cl | F | Öl |
| 8 | $-C_2H_5$ | $-C_2H_5$ | $-CH_3$ | Cl | Cl | Cl | Fp. 116 °C |

Anwendungsbeispiele

Beispiel A

Erysiphe-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine gute Wirksamkeit von 88 % Wirkungsgrad bei einer Wirkstoffkonzentration von 0,025 Gew.-% zeigt bei diesem Test z.B. die Verbindung gemäß Herstellungsbeispiel 2.

Beispiel B

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine gute Wirksamkeit zeigt bei diesem Test z.B. die Verbindung des Herstellungsbeispiels 2 bei einer Wirkstoffkonzentration von 0,025 Gew.-%.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine gute Wirksamkeit zeigen in diesem Test z.B. die Verbindungen gemäß den Herstellungbeispielen 1 und 2 bei einer Wirkstoffkonzentration von 0,025 Gew.-% in der Spritzbrühe.

Beispiel D

Botrytis-Test (Buschbohnen)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis

zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine gute Wirksamkeit zeigen in diesem Test z.B. die Verbindungen gemäß Herstellungsbeispielen 1 und 2 bei einer Wirkstoffkonzentration von 100 ppm.

## Ansprüche

1. N-Vinyl-3-cyano-4-phenyl-pyrrol-Derivate der allgemeinen Formel (I),

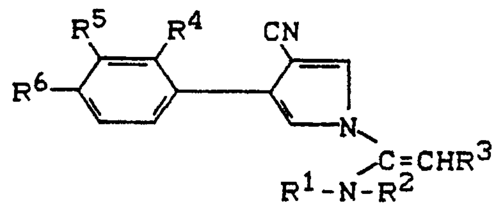

(I)

in welcher
$R^1$ für Alkyl steht,
$R^2$ für Alkyl steht oder
$R^1$ und $R^2$ gemeinsam mit dem Stickstoffatom, an welches sie gebunden sind, einen 5- bis 7-gliedrigen gesättigten, gegebenenfalls gleich oder verschieden, ein- bis mehrfach substituierten Ring bilden,
$R^3$ für Alkyl steht,
$R^4$ für Wasserstoff, Halogen, Alkyl, Halogenalkyl, Alkoxy, Halogenalkoxy, Alkylthio oder Halogenalkylthio steht,
$R^5$ für Wasserstoff, Halogen, Alkyl, Halogenalkyl, Alkoxy, Halogenalkoxy, Alkylthio oder Halogenalkylthio steht oder
$R^4$ und $R^5$ gemeinsam für durch Halogen substituiertes Alkylendioxy stehen und
$R^6$ für Wasserstoff oder Halogen steht.

2. N-Vinyl-3-cyano-4-pheny-lpyrrol-Derivate gemäß Anspruch 1, der Formel (I), in welcher
$R^1$ für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen steht oder
$R^1$ und $R^2$ gemeinsam mit dem Stickstoffatom, an welches sie gebunden sind, einen 5- bis 7-gliedrigen gesättigten, gegebenenfalls ein-bis fünffach, gleich oder verschieden durch Halogen, Alkyl mit 1 bis 4 Kohlenstoffatomen, Alkoxy mit 1 bis 4 Kohlenstoffatomen, Halogenalkyl oder Halogenalkoxy mit je 1 bis 4 Kohlenstoffatomen und 1 bis 9 gleichen oder verschiedenen Halogenatomen substituierten Ring bilden,
$R^3$ für geradkettiges oder verzweigtes Alkyl mit 1 bis 4 Kohlenstoffatomen steht,
$R^4$ für Wasserstoff, Halogen, geradkettiges oder verzweigtes Alkyl mit 1 bis 4 Kohlenstoffatomen, geradkettiges oder verzweigtes Halo genalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkylthio mit 1 bis 4 Kohlenstoffatomen, geradkettiges oder verzweigtes Halogenalkylthio mit 1 bis 4 Kohlenstoffatomen und 1 bis 9 gleichen oder verschiedenen Halogenatomen steht,
$R^5$ für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Halogenalkylthio mit 1 bis 4 Kohlenstoffatomen und 1 bis 9 gleichen oder verschiedenen Halogentomen steht,
oder
$R^4$ und $R^5$ gemeinsam für ein- oder zweifach durch Fluor substituiertes Methylendioxy stehen und
$R^6$ für Wasserstoff oder Fluor stehen.

3. N-Vinyl-3-cyano-4-phenyl-pyrrol-Derivate gemäß Anspruch 1 der Formel (I), bei welchen
$R^1$ für Methyl, Ethyl, n- oder iso-Propyl oder n-Butyl steht,

$R^2$ für Methyl, Ethyl, n- oder iso-Propyl oder n-Butyl steht oder
$R^1$ und $R^2$ gemeinsam mit dem Stickstoffatom, an welches sie gebunden sind, einen 5- bis 7-gliedrigen gesättigten, unsubstituierten Ring bilden,
$R^3$ für Methyl oder Ethyl steht,
$R^4$ für Wasserstoff, Fluor, Chlor, Brom, Methyl, Ethyl, Halogenalkyl mit 1 oder 2 Kohlenstoffatomen und 1 bis 5 gleichen oder verschiedenen Halogenatomen, insbesondere Fluor- und/oder Chloratomen, Methoxy, Ethoxy, Halogenalkoxy mit 1 oder 2 Kohlenstoffatomen und 1 bis 5 gleichen oder verschiedenen Halogenatomen, Methylthio, Ethylthio, Halogenalkylthio mit 1 oder 2 Kohlenstoffatomen und 1 bis 5 gleichen oder verschiedenen Halogenatomen, steht,
$R^5$ für Wasserstoff, Fluor, Chlor, Brom, Methyl, Ethyl, Halogenalkyl mit 1 oder 2 Kohlenstoffatomen und 1 bis 5 gleichen oder verschiedenen Halogenatomen, Methoxy, Ethoxy, Halogenalkoxy mit 1 oder 2 Kohlenstoffatomen und 1 bis 5 gleichen oder verschiedenen Halogenatomen, insbesondere Fluor- und/oder Chloratomen, Methylthio, Ethylthio, Halogenalkylthio mit 1 oder 2 Kohlenstoffatomen und 1 bis 5 gleichen oder verschiedenen Halogenatomen, steht,
oder
$R^4$ und $R^5$ gemeinsam für zweifach durch Fluor substituiertes Methylendioxy stehen und
$R^6$ für Wasserstoff oder Fluor steht.

4. N-Vinyl-3-cyano-4-phenyl-pyrrol-Derivate gemäß Anspruch 1 der Formel (I), in welcher
$R^1$ für Methyl oder Ethyl steht,
$R^2$ für Methyl oder Ethyl steht,
$R^3$ für Methyl oder Ethyl steht,
$R^4$ für Wasserstoff, Fluor, Chlor, Methyl, Ethyl oder Trifluormethyl steht,
$R^5$ für Wasserstoff, Chlor, Methyl oder Trifluormethyl steht und
$R^6$ für Wasserstoff oder Fluor steht.

5. Verfahren zur Herstellung von N-Vinyl-3-cyano-4-phenyl-pyrrol-Derivaten der Formel (I)

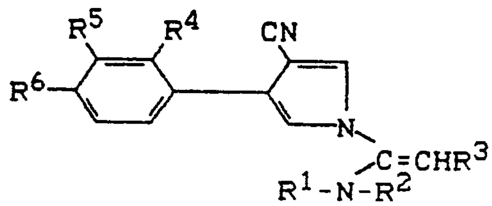

(I)

in welcher
$R^1$ für Alkyl steht,
$R^2$ für Alkyl steht oder
$R^1$ und $R^2$ gemeinsam mit dem Stickstoffatom, an welches sie gebunden sind, einen 5- bis 7-gliedrigen gesättigten, gegebenenfalls gleich oder verschieden, ein- bis mehrfach substituierten Ring bilden,
$R^3$ für Alkyl steht,
$R^4$ für Wasserstoff, Halogen, Alkyl, Halogenalkyl, Alkoxy, Halogenalkoxy, Alkylthio oder Halogenalkylthio steht,
$R^5$ für Wasserstoff, Halogen, Alkyl, Halogenalkyl, Alkoxy, Halogenalkoxy, Alkylthio oder Halogenalkylthio steht oder
$R^4$ und $R^5$ gemeinsam für durch Halogen substituiertes Alkylendioxy stehen, und
$R^6$ für Wasserstoff oder Halogen steht,
dadurch gekennzeichnet, daß man 3-Cyano-4-phenyl-pyrrol-Derivate der Formel (II)

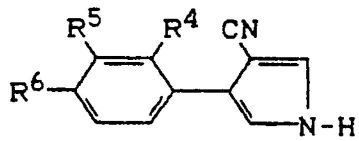

(II)

in welcher
$R^4$, $R^5$ und $R^6$ die oben angegebene Bedeutung haben,
mit 1-Aminoacetylen-Derivaten der Formel (III),

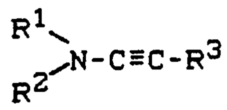

(III)

in welcher
$R^1$, $R^2$ und $R^3$ die oben angegebene Bedeutung haben,
gegebenenfalls in Gegenwart eines Verdünnungsmittels umsetzt.

6. Schädlingsbekämpfungsmittel, gekennzeichnet durch einen Gehalt an mindestens einem N-Vinyl-3-cyano-4-phenyl-pyrrol-Derivat der Formel (I) nach den Ansprüchen 1 und 5.

7. Verwendung von N-Vinyl-3-cyano-4-phenyl-pyrrol-Derivaten der Formel (I) nach den Ansprüchen 1 und 5 zur Bekämpfung von Schädlingen.

8. Verfahren zur Bekämpfung von Schädlingen, dadurch gekennzeichnet, daß man N-Vinyl-3-cyano-4-phenyl-pyrrol-Derivate der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man N-Vinyl-3-cyano-4-phenyl-pyrrol-Derivate der Formel (I) nach den Ansprüchen 1 und 5 mit Streckmitteln und/oder oberflächenaktiven Mitteln vermischt.

Europäisches Patentamt

# EUROPÄISCHER RECHERCHENBERICHT

Nummer der Anmeldung

**EP 90 11 1937**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 133 247 (CIBA-GEIGY AG)<br>* Ansprüche 1,7,9-12 *<br>- - - | 1,6-9 | C 07 D 207/34<br>C 07 D 405/04<br>A 01 N 43/36 |
| A | EP-A-0 281 731 (BAYER AG)<br>* Ansprüche 1,8-11 *<br>- - - | 1,6-9 | |
| A,D | EP-A-0 206 999 (CIBA-GEIGY AG)<br>* Ansprüche 1,7,9-15,17-19 *<br>- - - | 1,6-9 | |
| A | EP-A-0 310 558 (CIBA-GEIGY AG)<br>* Ansprüche 1,29,31-34 *<br>- - - | 1,6-9 | |
| A,D | EP-A-0 182 737 (CIBA-GEIGY AG)<br>* Ansprüche 1,8,15-18; Tabelle 3, Verbindung 3.178 *<br>- - - - - | 1,6-9 | |
| | | | **RECHERCHIERTE SACHGEBIETE (Int. Cl.5)** |
| | | | C 07 D 207/00<br>C 07 D 405/00<br>A 01 N 43/00 |

Der vorliegende Recherchenbericht wurde für alle Patentansprüche erstellt

| Recherchenort | Abschlußdatum der Recherche | Prüfer |
|---|---|---|
| Berlin | 08 Oktober 90 | VAN AMSTERDAM L.J.P. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument